# EUROPÄISCHE PATENTANMELDUNG

(11) **EP 1 264 575 A2**
(43) Veröffentlichungstag der Anmeldung: **11.12.2002**
(21) Anmeldenummer: 02012604.1
(22) Anmeldetag: 06.06.2002
(51) Int. Cl.: A61B 5/0408

(54) **Elektrodenanordnung**

(30) Priorität: 06.06.2001 DE 10127439
(71) Anmelder: PALMED GMBH, 89160 Dornstadt (DE)
(72) Erfinder: Feucht, Hans-Peter, 89134 Blaustein (DE); Harder, Albert Michael, 89160 Dornstadt (DE)
(74) Vertreter: Weber, Gerhard, Dipl.-Phys.

(57) **Zusammenfassung**

Zu einer Elektrodenanordnung für eine auf einer Hautfläche aufzulegende Elektrode werden ein vorteilhafter Aufbau eines Elektrodengehäuses und eine vorteilhafte Anordnung zur Fixierung einer Elektrode auf einen Körperteil angegeben, welche bevorzugt gemeinsam benutzt sind.

## Beschreibung

Die Erfindung betrifft eine Elektrodenanordnung für eine auf einer Hautfläche aufzulegende Elektrode.

Mittels elektrischer Signale können Körperfunktionen beeinflußt oder Körperparameter wie z. B. der Hautwiderstand gemessen werden. Hierzu können insbesondere elektrisch leitfähige Elektroden auf der Hautfläche angeordnet werden. Da bei den zum Einsatz kommenden Signalen nur sehr geringe Strom- und Spannungswerte ausgewertet werden, kommt dem Übergangswiderstand zwischen Elektrode und Haut eine besondere Bedeutung zu, wobei insbesondere eine geringe Variation dieses Übergangswiderstands im Verlauf einer Messung mit unveränderter Elektrodenposition wesentlich ist.

Aus der DE 17 41 748 U1 ist eine Saugelektrode mit einem hohlen Elektrodengehäuse bekannt, welches mit einer geschlossen ringförmigen Randfläche aus weichem, vorzugsweise klebendem Material auf einer Hautfläche aufgesetzt und durch Erzeugen von Unterdruck in dem Elektrodengehäuse zuverlässig auf der Hautfläche gehalten wird. Zusätzlich kann eine Fixierung des Elektrodengehäuses mittels um ein Körperteil zu einer Schlaufe schließbaren, an dem Elektrodengehäuse befestigten Bändern vorgesehen sein. Im Elektrodengehäuse ist eine Druckfeder mit einem Ende am Gehäuse und mit ihrem zweiten Ende an einer innerhalb der ringförmigen Randfläche befindlichen Elektrodenplatte befestigt und drückt diese gegen die Hautfläche. Eine Elektrodenzuleitung ist aus dem Gehäuse herausgeführt.

Die DE 88 08 120 U1 zeigt eine Tastelektrode mit einer unter dem Einfluß einer Federkraft freihändig auf eine Hautfläche anzusetzenden Spitzenelektrode, wobei durch eine integrierte Einrichtung zur Messung und Anzeige der Andruckkraft eine gute Reproduzierbarkeit von Messergebnissen erreicht werden soll.

In der DE 28 21 223 A1 ist ein medizinisches Meßgerät beschrieben, bei welchem ein Sensor und ein Wandler pneumatisch gekoppelt sind, um die Anordnung von Elektroden auf den Wandlerbereich und somit von dem am Patientenkörper angebrachten Sensor beabstandet zu verlegen. Der Sensor selbst besteht aus einer Meßkammer, welche luftdicht durch eine flexible Membran überspannt ist. Je nach Wölbung der Körperfläche, auf welche der Sensor aufgelegt ist, wird der Raum in der Meßkammer unterschiedlich verkleinert und somit Luft aus der Meßkammer in die ähnlich aufgebaute Wandlerkammer gedrückt, wo Elektroden zur Verschiebung einer Wandlermembran relativ zu einer festen Rückwand vorhanden sind. Ein engerer Bezug dieser Schrift zur vorliegenden Anmeldung ist nicht erkennbar.

In der DE 1514345 C3 ist eine Hautelektrode in Form einer Saugelektrode beschrieben, bei welcher eine flexible Membran auf der der Haut abgewandten Seite eines abstützenden Gehäuses angeordnet ist. Bei Erzeugen eines Unterdrucks in dem Elektrodengehäuse wird die Elektrode durch den äußeren Überdruck im Gehäuse nach unten auf die Haut gedrückt. In einer anderen Ausführung ist eine Stiftelektrode federbelastet gegen die Haut gedrückt, ohne daß eine Membran als Elektrodenträger vorgesehen ist.

Die US 3945384 zeigt eine mit einem Elektrolyten gefüllte Elektrode, welche durch ein Diaphragma einseitig abgeschlossen ist. Das Diaphragma ist mit dem Rand des den Elektrolyten enthaltenden Elektrodengehäuses verklebt. Im Inneren des Gehäuses ist eine Druckfeder angeordnet, welche die elektrische Verbindung zwischen einem elektrischen Anschlußkontakt und dem Elektrolyten bildet. Aus der DE 198 09 930 A1 ist es bekannt, gebräuchliche aufgeklebte Körperelektroden über ein flexibles Band mit integrierten elektrischen Leitern mit einem Basisgerät zum Verarbeiten und Auswerten der von den Elektroden gewonnenen Daten zu verbinden.

Der vorliegenden Erfindung liegt die Aufgabe zugrunde, eine weiter verbesserte Elektrodenanordnung mit auf eine Hautfläche aufzulegender Elektrode für zuverlässige Messungen anzugeben.

Erfindungsgemäße Lösungen sind in den unabhängigen Ansprüchen beschrieben. Die abhängigen Ansprüche enthalten vorteilhafte Ausgestaltungen und Weiterbildungen der Erfindung.

Durch die Überdeckung der von dem der Hautfläche zugewandten Gehäuserand aufgespannten Fläche durch eine flexible Membran, welche in einem zentralen Bereich die Elektrode trägt, kann auf einfache und kostengünstige Weise eine leicht handhabbare Elektrodenanordnung zur Verfügung gestellt werden, bei welcher die Andruckkraft der Elektrode auf die Hautfläche in dem Flächenbereich innerhalb der durch die Randfläche gebildeten Hautauflage des Elektrodengehäuses weitgehend unabhängig von der Andruckkraft des Elektrodengehäuses gegen die Hautfläche ist, so daß die Kontaktkraft, mit der die Elektrode auf der Hautfläche anliegt, im wesentlichen unabhängig von der Befestigung des Gehäuses und insbesondere über den Zeitverlauf einer Messung mit angelegter Elektrodenanordnung zumindest annähernd konstant ist. Die Verbindung von Elektrode und Gehäuse über die Kappe mit der flexiblen Membran kann zudem eine zumindest ungefähre Vorjustierung der Elektrode bezüglich des Gehäuses zum einen zentrierend in der von dem Gehäuserand aufgespannten Fläche und zum anderen in Richtung der Flächennormalen dieser Fläche, was nachfolgend auch als achsial oder Richtung einer Mittelachse der Elektrode bzw. Elektrodenanordnung bezeichnet ist, bewirken und damit die weitgehend problemlose Handhabung der Elektrode auch durch wenig geübte Personen gewährleisten.

Die Kappe bildet vorteilhafterweise eine flüssigkeitsdichte Abdeckung der von dem Gehäuserand umgebenden Gehäuseöffnung und verhindert das Eindringen von Flüssigkeit und Schmutz in das Gehäuseinnere, so daß die Elektrodenanordnung in dem hygienisch und messtechnisch bedeutenden, der Hautfläche zugewandten Bereich problemlos feucht gereinigt werden kann.

Die Kappe ist vorzugsweise zerstörungsfrei lösbar mit dem Gehäuserand verbunden, so daß in größeren Zeitintervallen die Elektrodenanordnung auch einer Intensivreinigung einschließlich des Gehäuseinnenraums unterzogen und/oder Kappe und Gehäuse unabhängig voneinander ersetzt werden können.

Die Kappe ist vorteilhafterweise formschlüssig mit dem Gehäuse verbunden, wobei der Formschluß günstigerweise eine Zentrierung bezüglich einer Mittelachse bewirkt und ein Verhaken an der Gehäusewand verhindert und/oder die Kappe in achsialer Richtung gegen die auf die Elektrode wirkende Federkraft in von der Hautfläche gelöster Situation der Elektrodenanordnung abfängt und zuverlässig auf dem Gehäuse hält.

Die flexible Membran besteht vorteilhafterweise aus einem elastischen, insbesondere gummielastischem Material, vorzugsweise Silikonkautschuk. Günstigerweise ist die gesamte Kappe einstückig materialhomogen aufgebaut und dadurch besonders kostengünstig herstellbar.

Die Membran ist vorzugsweise so geformt, daß die der Hautfläche zugewandte Membranfläche in spannungsfreiem Zustand gegen die Ebene der Randfläche in Richtung des Gehäuseinnenraums versetzt ist, insbesondere um ein Maß, das annähernd gleich der achsialen Dicke der vorzugsweise plattenförmigen, zwischen Membran und Hautfläche liegenden Elektrode ist, so daß die der Hautfläche zugewandte Elektrodenfläche in einem solchen spannungsfreien Zustand der flexiblen Membran zumindest annähernd in der Ebene der Randfläche liegt.

In einer typischen Messituation nimmt die flexible Membran damit einen weitgehend spannungsfreien Zustand ein und trägt nicht zur Kontaktkraft, mit welcher die Elektrode auf die Hautfläche drückt, bei, so daß diese Kontaktkraft allein durch eine Feder bestimmbar und auch bei Serienfertigung der Elektrodenanordnung gleichmäßig vorgebbar ist. Der Abstand zwischen der Elektrode und der Randfläche beträgt radial bezüglich einer Mittelachse vorteilhafterweise wenigstens 20 % des Elektrodenradius, so daß dieser Abstand als Spielraum der Elektrode bei Bewegung in achsialer Richtung oder auch bei leichter Verkippung mit geringem Verformungswiderstand zur Verfügung steht und eine Anpassung der Elektrodenposition an den Verlauf der Hautfläche ermöglicht, ohne daß die Kontaktkraft wesentlich beeinflußt wird. In der Situation mit von der Hautfläche gelöster Elektrodenanordnung ist die flexible Membran durch die Federkraft vorgespannt nach außen gewölbt.

Der Innenraum des Elektrodengehäuses ist vorteilhafterweise gegen die Umgebung druckausgeglichen, vorzugsweise über eine der Hautfläche und der Kappe abgewandte bzw. beabstandete Öffnung.

Die zwischen flexibler Membran und Hautfläche liegende Elektrode ist vorteilhafterweise durch die Membran ins Gehäuseinnere durchkontaktiert, wozu vorzugsweise in der Membran eine zentrale oder mehrere um die Mittelachse gruppierte Öffnungen und auf der der Hautfläche ab- und der Membran zugewandten Seite der Elektrode von dieser an entsprechenden Positionen ein oder mehrere Stifte abstehen und durch die Öffnung(en) hindurchragen. Hierdurch wird eine zuverlässige Zentrierung der Elektrode gewährleistet und eine Beschädigung der Membran wie z. B. bei Befestigung mittels eines durch eine geschlossene Membran durchgedrückten Dorns vermieden. Günstigerweise ist auf der der Hautfläche abgewandten Seite der flexiblen Membran ein Gegenelement vorgesehen, welches mit dem bzw. den durch die Membran ragenden Stiften korrespondiert und eine Verspannung der Membran zwischen Elektrode und Gegenelement ermöglicht. Vorzugsweise ist das Gegenelement platten- oder ringförmig, so daß eine große positionsstabilisierende und dichtende Anlagefläche der Membran an der Elektrode und/oder dem Gegenelement gegeben ist. Alternativ oder zusätzlich kann auch eine positionierende und dichtende Wirkung durch ein geringes Untermaß der Öffnungen in der Membran gegenüber den Stiften vorliegen. Besonders einfach und günstig ist eine Ausführung mit nur einem Stift auf der Elektrode und einer zentralen Öffnung in der Membran und einem Gegenelement, welches auf den Stift befestigbar, z. B. auf ein Gewinde des Stifts aufschraubbar ist.

Zur Aufbringung der Kontaktkraft ist vorteilhafterweise wie an sich bekannt eine Druckfeder einsetzbar, welche zugleich als elektrische Zuleitung von einem Anschlußkontakt zur Elektrode dienen kann. Besonders vorteilhaft ist eine Anordnung, bei welcher die Feder lediglich klemmend und/oder unter dem Einfluß der Federkraft an dem Anschlußkontakt und der Elektrode oder einem weiteren im elektrischen Zuleitungsweg liegenden Element wie dem beschriebenen Gegenelement anliegt und aufwendige Verbindungstechniken wie Verschrauben, Vernieten, Schweißen, Löten etc. vermieden werden. Die Oberflächen von leitenden Elementen der Elektrodenanordnung im elektrischen Signalweg sind vorzugsweise korrosionsgeschützt und niederohmig, insbesondere vergoldet.

Zur Befestigung eines Elektrodengehäuses auf einer Hautoberfläche ist eine Anordnung von Vorteil, bei welcher ein erstes Band als eine Manschette um ein Körperteil geschlungen ist und vorzugsweise unter leichter Zugspannung steht. Da die Zugspannung beim Anlegen im Regelfall nicht präzise einstellbar ist und sich durch Stauungen, Anschwellen etc. von Körpergewebe im Verlauf einer Messung auch verändern kann, ist bei bekannten Anordnungen, bei welchen die Anpresskraft des Elektrodengehäuses auf die Hautfläche im wesentlichen durch diese Zugkraft in der Manschette bestimmt ist, die Anpresskraft starken Schwankungen unterworfen. Die Kontaktkraft, mit welcher die Elektrode auf die Hautfläche drückt, ist auch in einem solchen Fall durch die vorstehend ausführlich beschriebene Elektrodenanordnung weitgehend gleichmäßig. Eine alternative oder zusätzliche Maßnahme zur Erzielung einer gleichmäßigen Kontaktkraft ist dadurch gegeben, daß in dem als Manschette um das Körperteil geschlungenen ersten Band eine Aussparung vorgesehen ist, durch welche die Elektrode nach Anlegen der Manschette auf die Haut auflegbar ist, und daß ein zweites Band die Aussparung und eine in diese eingesetzte Elektrode bzw. ein Elektrodengehäuse zwischen gegenüberliegenden Befestigungspunkten des ersten Bandes überbrückt und die Elektrode durch dieses zweite Band in der Aussparung des ersten Bandes fixiert ist. Das zweite Band ist vorzugsweise elastisch dehnbar, wogegen das erste Band in dem Bereich zwischen den Befestigungspunkten der Lasche nicht dehnbar ist.

Das zweite Band kann an den Befestigungspunkten fest mit dem ersten Band verbunden sein, wobei das Einsetzen einer Elektrode unter elastischem Wegziehen des zweiten Bandes über der Aussparung erfolgen kann. In anderer bevorzugter Ausführung ist das zweite Band als Lasche nur einseitig mit dem ersten Band fest und in einem gegenüberliegenden Befestigungspunkt lösbar mit dem ersten Band verbunden. Unter Band sei dabei nicht nur streng ein gleichmäßig breiter Streifen verstanden sondern allgemein ein die entsprechende Funktion erfüllendes Haltemittel, welches auch in mehrere an unterschiedlichen Punkten ansetzende Teile unterteilt sein kann.

Die Kraft, mit welcher das Elektrodengehäuse auf die Hautfläche in dem Ausschnitt des ersten Bandes drückt, ist damit vorteilhafterweise von den Kräften, welche das erste Band am Körper fixieren, entkoppelt und im wesentlichen nur durch die Spannkraft des zweiten Bandes bestimmt. Der Bereich dieser Spannkraft ist bei beidseitig fest mit dem ersten Band verbundenem zweitem Band mit geringer Variation vorgegeben und kann auch bei nur einseitig fest verbundener Lasche durch Einschränkung des Verstellbereichs der Befestigung des freien Laschenendes, insbesondere auf weniger als 10 % der Laschenlänge oder weniger als 30 % der elastischen Dehnbarkeit der Lasche, gering gehalten werden.

Besonders vorteilhaft ist eine Elektrodenanordnung, bei welcher ein Elektrodengehäuse mit flexibler Membran durch eine aus zwei Bändern bestehende Halterung in der beschriebenen Art auf eine Hautfläche gedrückt ist.

Die Erfindung ist nachfolgend anhand bevorzugter Ausführungsbeispiele unter Bezugnahme auf die Abbildungen noch eingehend veranschaulicht. Dabei zeigt
- Fig. 1: Einzelteile eines Aufbaus einer Elektrodenanordnung
- Fig. 2: eine aus den Einzelteilen nach Fig. 1 zusammengesetzte Elektrodenanordnung
- Fig. 3: eine um 90° gedrehte Ansicht zu Fig. 2
- Fig. 4: eine Elektrodenanordnung an einem Körperteil
- Fig. 5: ein in Fig. 4 benutztes zweifaches Band
- Fig. 6: das Band nach Fig. 5 in Draufsicht auf die Bandfläche

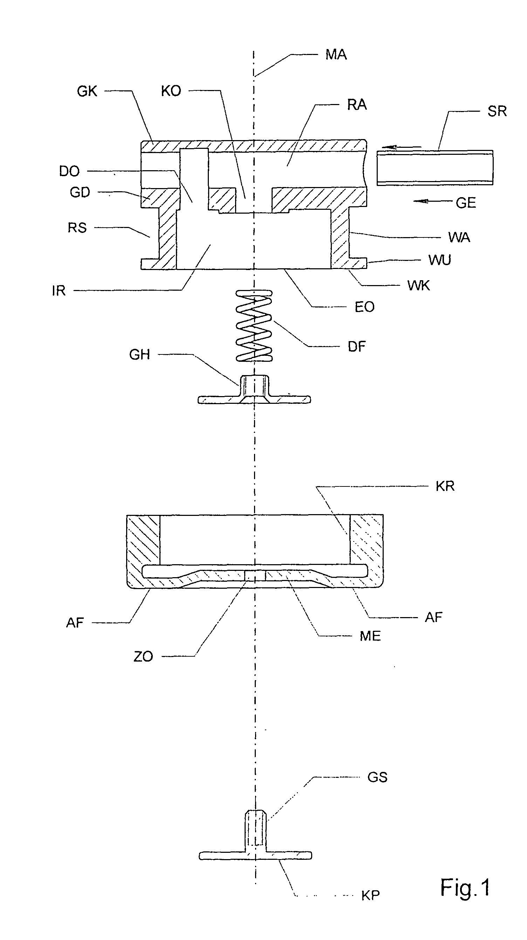

In Fig. 1 sind die Einzelteile eines bevorzugten Aufbaus einer Elektrodenanordnung vor dem Zusammenbau als Schnittbild skizziert. Ein vorzugsweise einstückiges Elektrodengehäuse GE umschließt mit einem Gehäusedeckel und einer ringförmigen Gehäusewand WA einen Gehäuse-Innenraum IR. Die Wand WA verläuft vorzugsweise kreisringförmig um eine Mittelachse MA. Der Innenraum IR ist zu der vom Gehäusedeckel abgewandten Seite hin offen. Die Wand WA ist in achsparalleler Richtung durch einen umlaufenden Einzug RS abgestuft. Die dem Gehäusedeckel abgewandte und einen radial nach außen weisenden Wulst tragende Wandkante WK liegt in einer Gehäuseebene EO.

Das Gehäuse setzt sich über dem Gehäusedeckel in einem Griffkörper GK, welcher in der skizzierten Ausführungsform nicht drehsymmetrisch um die Mittelachse MA ist, fort und umfaßt eine quer zur Mittelachse verlaufende Rohraufnahme RA für ein in diese klemmend einsetzbares Kontaktrohr SR. Vom Rohrinnenraum führt eine Kontaktöffnung zu der Rohraufnahme und eine Druckentlastungsöffnung DO zur Umgebung.

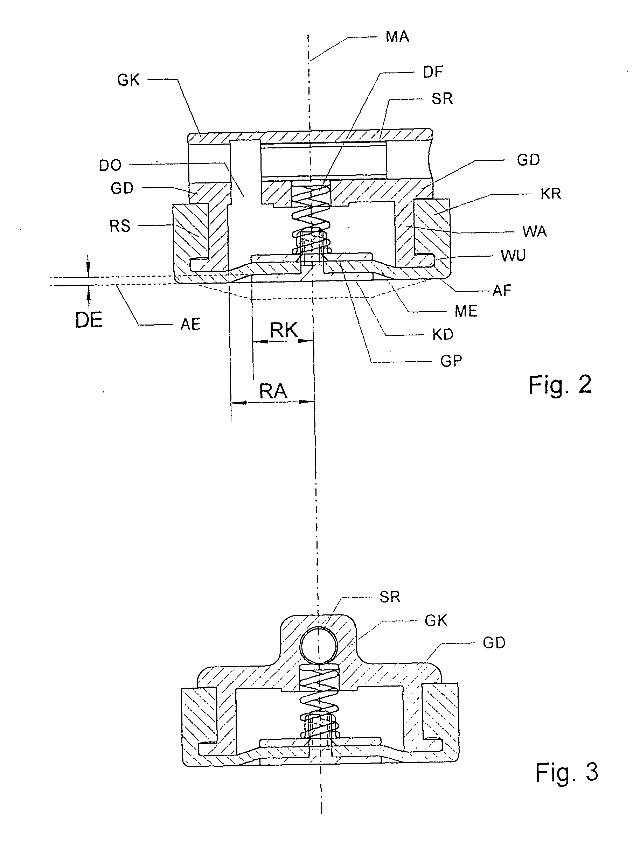

Eine Kappe KA aus gummielastischem Material, insbesondere Silikonkautschuk ist der Form der Gehäusewand WA, vorzugsweise achsial und/oder radial mit geringem Untermaß, angepaßt und weist insbesondere eine flexible Membran ME auf, welche quer zur Mittelachse MA liegt und sich als eine Ringwand RW vom Rand der Membran in achsialer Richtung fortsetzt. Die Ringwand weist einen radial nach innen gerichteten Kragen KR auf, welcher im zusammengesetzten Zustand nach Fig. 2 in dem Einzug RS der Gehäusewand einliegt und durch den Wulst WU achsial hintergriffen ist. Die Ringwand RW der Kappe KA ragt radial über das Gehäuse GE hinaus und kann daher zum Abnehmen ohne Werkzeug von Hand gegriffen und über den Wulst WU gezogen werden.

Die flexible Membran ist in der skizzierten spannungsfreien Form gegen eine durch eine radial außen liegende ringförmige Randfläche AF als Auflagefläche der Membran ME definierte Auflageebene AE in einem radial innen liegenden Bereich um ein Maß DE achsial in Richtung des Innenraums zurückgesetzt. Sie verläuft in diesem inneren Bereich vorzugsweise eben und senkrecht zur Mittelachse MA. Die Membran weist bei der Mittelachse eine zentrale Öffnung ZO auf.

Die Elektrode ist als ebene Kontaktplatte KP ausgeführt, welche an ihrer der flexiblen Membran zugewandten Seite einen Gewindestift GS trägt, welcher durch die zentrale Öffnung der flexiblen Membran durchgesteckt wird und mit der der Membran abgewandten Kontaktfläche auf der Hautfläche aufliegt. Mittels des Gewindestifts und eines Gegenelements, im skizzierten Beispiel einer Gegenplatte mit einer Gewindehülse, wird die Kontaktplatte an der flexiblen Membran befestigt, wobei eine weitgehend flüssigkeitsdichte Abdichtung zur Auflageebene hin durch das Einklemmen der Membran zwischen Kontaktplatte und Gegenplatte und/oder durch ein geringes Untermaß der zentralen Öffnung ZO in der Membran gegenüber dem Gewindestift erreicht wird. Die Elektrode kann auch einstückig mit zwei durch einen radialen umlaufenden Einstich beabstandeten Platten ausgeführt sein und in eine zentrale Öffnung der Membran eingeknöpft werden. Die Dicke der Kontaktplatte KP ist vorzugsweise gleich oder geringfügig kleiner als der achsiale Versatz DE des zentralen Bereichs der Membran gegen die Auflageebene AF, so daß bei auf eine Hautfläche aufgesetzter Elektrodenanordnung und in der Ebene der Auflagefläche liegender Kontaktfläche der Kontaktplatte die Membran nicht elastisch verformt ist.

Eine gewendelte Druckfeder DF aus leitendem Material verbindet in zusammengebautem Zustand elektrisch die Elektrode mit dem Kontaktrohr SR, indem sie mit einem Ende durch die Kontaktöffnung KO des Gehäusedeckels an dem Kontaktrohr SR und mit dem anderen Ende an der metallischen Gegenplatte GP, insbesondere unter klemmender Umfassung der Gewindehülse anliegt und dabei zusammengedrückt vorgespannt ist. Durch die Vorspannung der Druckfeder ist die Membran ME nach außen vorgewölbt, wie in Fig. 2 mit unterbrochener Linie eingezeichnet ist.

Die Elektrodenanordnung wird durch Krafteinwirkung auf das Gehäuse gegen eine Hautfläche gedrückt und stützt sich mit der in achsialer Verlängerung der Gehäusewand liegenden ringförmigen Auflagefläche AF auf der Hautfläche ab. Der Innenradius dieser Auflagefläche ist in Fig. 2 mit RA bezeichnet. Der Außenradium der Kontaktplatte KP sei RK. Vorzugsweise ist RA ≥ 1,2 RK, so daß die flexible Membran in dem Übergangsbereich von der Auflagefläche zu dem zentralen Plattenbereich einerseits wenig die Andruckkraft der Kontakplatte verfälschenden Verformungswiderstand gegen geringe achsiale Verschiebungen und/oder Verkippungen der Kontaktplatte bietet und andererseits hinreichend dick ausgeführt sein kann, um mechanischen Belastungen im gewöhnlichen Gebrauch und insbesondere auch der Auswölbung durch die Federkraft standzuhalten.

In Fig. 3 ist die Elektrodenanordnung nach Fig. 2 in einer um 90° um die Mittelachse gedrehten geschnittenen Ansicht mit Blick in Richtung der Längsachse des Kontaktrohrs SR, welches als Steckbuchse für eine wegführende elektrische Leitung dient, gezeichnet.

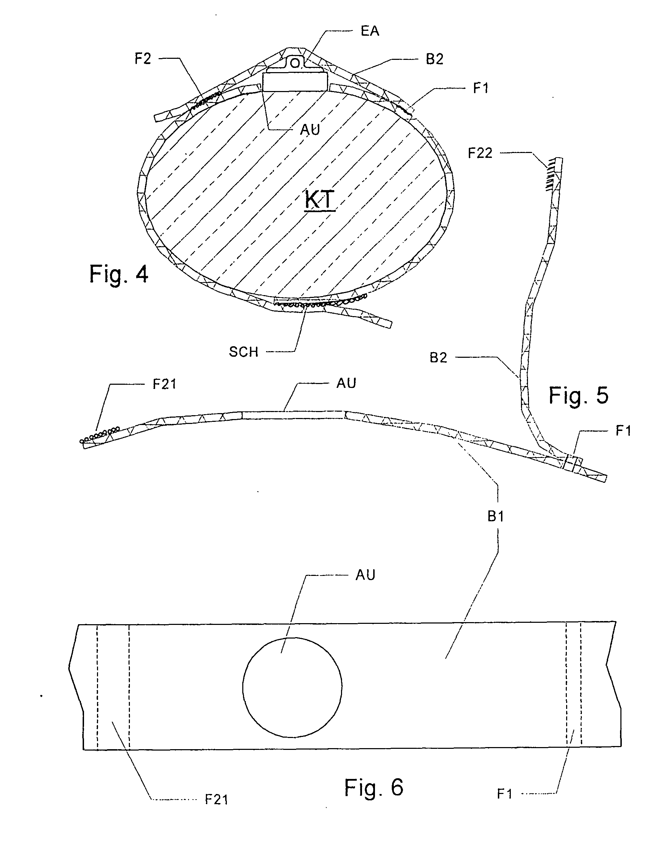

In Fig. 4 ist eine Anordnung skizziert, bei welcher ein erstes Band B1 als Manschette um ein Körperteil KT geschlungen und an einen Verschluß, z. B. einem Klettverschluß zu einer Schleife geschlossen ist. Das erste Band B1 wird unter leichter Zugspannung geschlossen, um einen sicheren Sitz auf dem Körperteil zu erreichen. In dem ersten Band ist eine Aussparung AU vorgesehen, durch welche eine Elektrodenanordnung EA, vorzugsweise eine Elektrodenanordnung nach Fig.1 bis Fig. 3 mit Gehäuse GE und Kappe KA auf die Haut des Körperteils KT aufgesetzt werden kann.

Die Elektrodenanordnung ist von einem zweiten Band B2 überspannt, welches an bezüglich der Aussparung AU gegenüberliegenden Befestigungsstellen F1 und F2 mit dem ersten Band B1 verbunden ist. Vorzugsweise ist das Band B1 als Lasche an einer Befestigungsstelle F1 unlösbar und damit unverlierbar und an der weiteren Befestigungsstelle F2 lösbar, z. B. über einen Haken- oder Klettverschluß mit Verschlußelementen F21 auf Seiten des ersten und F22 auf Seiten des zweiten Bandes, mit dem zweiten Band verbunden. Das zweite Band ist vorteilhafterweise zumindest über einen Abschnitt elastisch dehnbar ausgeführt.

Das zweite Band steht in der Anordnung nach Fig. 4 unter leichter Zugspannung, durch welche ein zuverlässiger Kontakt der Elektrode mit der Hautfläche gewährleistet ist. Bei einseitig lösbarer Verbindung B2 und einer Möglichkeit unterschiedlicher Befestigungsposition in Längsrichtung des zweiten Bandes, wie dies z. B. für Klettverschlüsse typisch ist, ist die Zugkraft im zweiten Band und damit die Anpresskraft des Gehäuse in Richtung der Hautkraft veränderlich und von der beim Schließen der Lasche ausgeübten Zugkraft abhängig. Der Variationsbereich der Zugkraft im zweiten Band kann gering gehalten werden, indem der Verstellbereich der Befestigungsposition von F22 relativ zu F21 gering gehalten ist und insbesondere klein, vorzugsweise geringer als 30 % der elastischen Längendehnbarkeit des zweiten Bandes ist. Bei beidseitig in F1 und F2 fest verbundenem zweiten Band wird dieses zum Einsetzen der Elektrodenanordnung unter elastischer Dehnung über der Aussparung abgehoben und drückt nach Einsetzen der Elektrodenanordnung diese gegen die Hautfläche.

Die Fig. 5 zeigt einen Abschnitt des ersten Bandes mit geöffnetem zweiten Band. In Fig. 6 ist das erste Band in Draufsicht auf die Aussparung mit in der Zeichenebene hinten liegendem zweiten Band dargestellt. Die Bänder sind nicht zwingend als gleichmäßig breite Streifen ausgeführt, sondern können auch anders strukturiert sein. Insbesondere kann das zweite Band z. B. eine an die Elektrodenanordnung angepaßte Struktur aufweisen und/oder an mehr als zwei Befestigungsstellen mit dem ersten Band verbunden sein. Ein Band zum Andrücken der Elektrode auf die Hautfläche kann auch an dem Elektrodengehäuse eingehängt sein.

Die vorstehend und die in den Ansprüchen angegebenen sowie die den Abbildungen entnehmbaren Merkmale sind sowohl einzeln als auch in verschiedener Kombination vorteilhaft realisierbar. Die Erfindung ist nicht auf die beschriebenen Ausführungsbeispiele beschränkt, sondern im Rahmen fachmännischen Könnens in mancherlei Weise abwandelbar. Die Elektrodenanordnungen nach Fig. 1 bis Fig. 3 einerseits und nach Fig. 4 bis Fig. 6 andererseits sind vorzugsweise gemeinsam realisiert.

## Patentansprüche

1. Elektrodenanordnung mit einer elektrisch leitenden Elektrode und einem Gehäuse, wobei das Gehäuse mittels Haltemitteln gegen eine Hautfläche drückbar ist und sich dabei mit einem der Hautfläche zuweisenden Gehäusewandabschnitt einer die Elektrode umgebenden Randfläche abstützt, und wobei die Elektrode relativ zum Gehäuse verschiebbar und bei auf der Hautfläche aufliegender Randfläche mit einer Kontaktkraft gegen die Hautfläche gedrückt ist, **dadurch gekennzeichnet, daß** eine der Hautfläche zugewandte Kappe mit dem Gehäusewandabschnitt verbunden ist und die von diesem aufgespannte Fläche mit einer flexiblen Membran überbrückt, welche in einem zentralen Bereich innerhalb der Randfläche die Elektrode trägt.

2. Anordnung nach Anspruch 1, **dadurch gekennzeichnet, daß** die Kappe formschlüssig und lösbar mit dem Gehäuse verbunden ist.

3. Anordnung nach Anspruch 2, **dadurch gekennzeichnet, daß** der Formschluß der Verbindung zentrierend auf die Position der Elektrode innerhalb der Randfläche und/oder haltend gegen in Richtung der Flächennormalen der flexiblen Membran gerichtete abziehende Kräfte wirkt.

4. Anordnung nach einem der Ansprüche 1 bis 3, **dadurch gekennzeichnet, daß** die Elektrode durch die Membran ins Gehäuseinnere durchkontaktiert ist.

5. Anordnung nach Anspruch 4, **dadurch gekennzeichnet, daß** die Membran wenigstens eine Öffnung aufweist, durch welche ein Stift der Elektrode ins Gehäuseinnere hindurchragt.

6. Anordnung nach einem der Ansprüche 1 bis 5, **dadurch gekennzeichnet, daß** die Membran zwischen der Elektrode auf der Seite der Hautfläche und einem Gegenelement im Gehäuseinneren verspannt ist.

7. Anordnung nach einem der Ansprüche 1 bis 6, **dadurch gekennzeichnet, daß** die Kontaktkraft von einer Druckfeder im Gehäuseinneren aufgebracht ist.

8. Anordnung nach Anspruch 7, **dadurch gekennzeichnet, daß** die Druckfeder elektrisch leitend ist und als elektrische Verbindung zwischen einem Anschlußkontakt und der Elektrode dient.

9. Anordnung nach Anspruch 8, **dadurch gekennzeichnet, daß** die Kontaktierung der Druckfeder auf Seiten der Elektrode und auf Seiten des Anschlußkontakts nur durch Anliegen unter der Federkraft und/oder durch elastische Klemmung erfolgt.

10. Anordnung nach einem der Ansprüche 1 bis 9, **dadurch gekennzeichnet, daß** der Abstand zwischen der Elektrode und der diese umgebenden Randfläche radial bezüglich einer Mittelachse wenigstens 20 % des Radius der Elektrode beträgt.

11. Anordnung nach einem der Ansprüche 1 bis 10, **dadurch gekennzeichnet, daß** die Membran aus elastisch verformbarem, insbesondere gummielastischem Material besteht.

12. Anordnung nach einem der Ansprüche 1 bis 11, **dadurch gekennzeichnet, daß** die Kappe einstückig materialhomogen ausgeführt ist.

13. Anordnung nach einem der Ansprüche 1 bis 12, **dadurch gekennzeichnet, daß** die Membran in unverformtem Zustand im Bereich der Elektrode zum Gehäuseinneren hin gegen die Randfläche versetzt ist.

14. Anordnung nach einem der Ansprüche 1 bis 13, **dadurch gekennzeichnet, daß** im unverformten Zustand der Membran die Auflagefläche der Elektrode in der Ebene der Randfläche liegt.

15. Anordnung nach einem der Ansprüche 1 bis 14, **dadurch gekennzeichnet, daß** die Oberflächen von Elektrode und/oder Druckfeder und/oder Anschlußkontakt zumindest teilweise vergoldet sind.

16. Anordnung nach einem der Ansprüche 1 bis 15, **dadurch gekennzeichnet, daß** die Kappe das Gehäuseinnere flüssigkeitsdicht abschließt und wasserabweisend ist.

17. Anordnung nach einem der Ansprüche 1 bis 16, **dadurch gekennzeichnet, daß** das Gehäuseinnere über eine der Kappe abgewandte Öffnung druckausgeglichen zur Umgebung ist.

18. Elektrodenanordnung mit einer ein Körperteil umschlingenden Manschette, welche ein Elektrodengehäuse mit einer Elektrode gegen eine Hautfläche drückt, **dadurch gekennzeichnet, daß** die Manschette aus einem ersten Band, welches das Körperteil umschlingt und eine Aussparung über der Hautfläche für die Elektrode aufweist und einer die Aussparung zwischen gegenüberliegenden Befestigungspunkten des ersten Bandes überbrückenden Lasche.

19. Anordnung nach Anspruch 18, **dadurch gekennzeichnet, daß** die Lasche an einem Ende fest und an einem anderen Ende lösbar mit dem ersten Band verbunden ist.

20. Anordnung nach Anspruch 18 oder 19, **dadurch gekennzeichnet, daß** die Lasche elastisch dehnbar ist.

21. Anordnung nach einem der Ansprüche 18 bis 20, **dadurch gekennzeichnet, daß** das erste Band im Bereich der Aussparung nicht dehnbar ist.
